Europäisches Patentamt

European Patent Office

Office européen des brevets

(19)

(11) Publication number : **0 526 258 A2**

(12) **EUROPEAN PATENT APPLICATION**

(21) Application number : 92307089.0

(22) Date of filing : 03.08.92

(51) Int. Cl.[5] : **C12P 1/06,** C12N 1/20,
// A61K35/74 , (C12P1/06,
C12R1:01), (C12N1/20,
C12R1:01)

(30) Priority : 28.05.92 US 889722
01.08.91 US 738969

(43) Date of publication of application :
03.02.93 Bulletin 93/05

(84) Designated Contracting States :
AT BE CH DE DK ES FR GB GR IE IT LI LU NL
PT SE

(71) Applicant : ELI LILLY AND COMPANY
Lilly Corporate Center
Indianapolis Indiana 46285 (US)

(72) Inventor : Yao, Raymond Che-Fong
1189 Woodgate Drive
Carmel, Indiana 46032 (US)
Inventor : Colacino, Joseph Matthew
9415 Moorings Boulevard
Indianapolis, Indiana 46256 (US)

Inventor : Debono, Manuel
5257 Hinesley Avenue
Indianapolis, Indiana 46208 (US)
Inventor : Molloy, Robert Michael, Sr.
603 North 50 East
Danville, Indiana 46122 (US)
Inventor : Tang, Joseph Chiou-chung
1512 Dorchester Place
Carmel, Indiana 46033 (US)
Inventor : Berry, Dennis Randolph
827 Briarwood Drive
Greenwood, Indiana 46142 (US)
Inventor : Dantzig, Anne Hollins
18 Twin Oaks
Crawfordsville, Indiana 47933 (US)
Inventor : Hamill, Robert L.
617 Brookview Drive
Greenwood, Indiana 46142 (US)

(74) Representative : Hudson, Christopher Mark et
al
Erl Wood Manor
Windlesham Surrey GU20 6PH (GB)

(54) A87689 Compounds, their production and use as phospholipase-A2 inhibitors.

(57) Fermentation product A87689 is produced by a strain of Amycolatopsis mediterranei selected from NRRL 18815 and NRRL 18851, or an A87689-producing mutant thereof. A87689, its $C_{1-6}$-alkyl ether and $C_{1-6}$-alkanoyl ester derivatives and pharmaceutically acceptable salts thereof are phospholipase $A_2$ inhibitors that are useful as antiinflammatory agents. Fermentation products known as BU-3889V, its components and pharmaceutically acceptable salts thereof, are phospholipase $A_2$ inhibitors that are also useful as antiinflammatory agents. A87689 compounds, including its $C_{1-6}$-alkyl ether and $C_{1-6}$-alkanoyl derivatives, used alone or in combination with nucleoside reverse transcriptase inhibitors, non-nucleoside reverse transcriptase inhibitors and HIV protease inhibitors, are also useful for managing retroviral infections in mammals and are particularly useful for managing HIV and/or AIDS in humans.

EP 0 526 258 A2

The present invention relates to a novel quartromicin, its derivatives and pharmaceutically acceptable salts thereof, and processes for enzyme inhibition and antiviral activity.

More particularly, this invention includes a novel phospholipase $A_2$ ($PLA_2$) inhibitor, A87689, which can be isolated in substantially pure form from fermentation cultures of Amycolatopsis mediterranei. This invention further includes processes for treating retroviral infections, particularly HIV (and the treatment of the resulting Acquired Immune Deficiency Syndrome (AIDS)), and oncovirinae such as human T-cell leukemia virus, and the like. The present invention also relates to pharmaceutical formulations containing A87689 compounds, and methods of use of the present compounds alone, or in combination with other agents, for the inhibition of $PLA_2$ and for the management of retroviral infections.

Inflammatory disorders account for a significant amount of debilitating diseases. Inflammatory tory states, such as arthritis, psoriasis, asthma, and possibly atherosclerosis, stem from inflammatory reactions in the joints, skin, and blood vessels. It is generally believed that a central role in the inflammatory reaction is the production of phospholipid metabolites called eicosanoids. The eicosanoids represent a family of important mediators such as the leukotrienes, prostaglandins, lipoxins, hydroxyeicosatetranoic acid, and thromboxanes. It is believed that the generation of eicosanoids is dependent on the availability of arachidonic acid which is liberated from phospholipids by the action of phospholipase $A_2$ ($PLA_2$).

$PLA_2$ is the common name for phosphatide 2-acylhydrolase, which catalyzes the hydrolysis of the sn-2-acyl ester bond of phosphoglycerides which results in the production of equimolar amounts of lysophospholipids and free fatty acids [Dennis, E-A.,The Enzymes, Vol. 16, Academic Press, New York, (1983)]. $PLA_2$ enzymes are found in all living species and form a diverse family of enzymes. Over forty $PLA_2$ enzymes have been structurally characterized, and they show a high degree of sequence homology. [J. Chang, J. H. Musser, and H. McGregor, "Phospholipase $A_2$: Function and Pharmacological Regulation," Biochem. Pharm. 36: 2429-2436, (1987)].

The best characterized varieties of $PLA_2$ enzyme are the secreted forms, which are released into the extracellular environment where they aid in the digestion of biological materials. The secreted forms have a mw of about 12-15,000 (Chang, et al, supra). In contrast, cytosolic $PLA_2$s are found in small amounts within the cell and play a key role in the biosynthetic pathway leading to the formation of the platelet activating factors and the eicosanoids. [Mobilio, D. and Marshall, L.A., "Recent Advances in the Design and Evaluation of Inhibitors of Phospholipase $A_2$," Ann. Reports in Med. Chem. 24; 157-166, (1989)]. The cytosolic $PLA_2$s have a mw of approximately 85,000. [Clark, J.D., Lin, L.L., Kriz, W., Ramesha, C.S., Sultzman, Lin, A.Y., Merlona, N., Knots, J.L., Cell 65: 1043-1051 (1991)]. Free arachidonic acid is the rate limiting precursor for the production of eicosanoids and is liberated from its membrane phospholipid store by the action of cytosolic $PLA_2$. [Dennis, E. A., "Phospholipase $A_2$ Mechanism: Inhibition and Role in Arachidonic Acid Release," Drug Dev. Res.10: 205-220, (1987)]. The same enzymatic step also produces lysophospholipids which may be converted to form platelet-activating factors. Thus, it is believed that cytosolic $PLA_2$ is central to the regulation of the biosynthetic pathways of potent lipid mediators of inflammation.

Due to the central role in the inflammation process that is played by cytosolic $PLA_2$, it is desirable to identify and characterize new inhibitors of the enzyme- Such inhibitors may lead to new therapies for the treatment of arthritis, asthma, atherosclerosis, and inflammatory diseases of the skin (e.g. psoriasis) and the bowel (e.g. irritable bowel syndrome). Identification of such specific $PLA_2$ inhibitors may aid the medical community in reaching the goal of control of inflammatory disease states.

Since the isolation of the first human retrovirus in 1980, human T-leukemia virus (HTLV, now HTLV-1), a substantial body of knowledge has been accumulated. Retroviruses have been associated with many diseases including rapid and long-latency malignancies, wasting diseases, neurological disorders, immunodeficiencies, as well as lifelong viremia in the absence of any obvious ill effects. See, e.g. Fundamental Virology (Fields, B.N., et al., eds., 1991). The viruses classified in the family retroviridae have been taxonomically subdivided into three distinct subfamilies, oncovirinae, lentivirinae and spumavirinae. The subfamily oncovirinae most notably includes the human T-cell leukemia virus. The subfamily spumavirinae, otherwise known as the "foamy" viruses, include many human and primate isolates (e.g., Simian foamy virus), but are generally considered to be benign. The third subfamily of the family retroviridae, lentivirinae, includes human immunodeficiency virus (HIV).

More particularly, HIV (also referred to as the AIDS virus) was previously known as, or referred to as, LAV, HTLV-III, or ARV, and is now designated HIV-1. Other closely related variants of HIV-1 include HIV-2 and SIV (simian immunodeficiency virus). The use of the term "HIV" throughout this specification includes HIV-1 and HIV-2 unless otherwise specified.

The complex disease, acquired immunodeficiency syndrome (AIDS), includes progressive destruction of the immune system and degeneration of the central and peripheral nervous system. HIV appears to preferentially attack helper T-cells (T-lymphocytes or OKT4-bearing T-cells) and also other human cells, e.g., certain cells within the brain. The helper T-cells are invaded by the virus and the T-cell becomes an HIV virus producer. The helper T-cells are quickly destroyed and their number in a human body is depleted to such an extent that the body's B-cells, as well as other T-cells normally stimulated by helper T-cells, no longer function normally or produce sufficient lymphokines and antibodies to destroy the invading virus or other invading microbes.

While HIV, per se, does not necessarily cause death,it does cause the human's immune system to be so severely depressed that the human falls prey to various other diseases such as herpes, cytomegalovirus, Kaposi's sarcoma, and Epstein-Barr virus-related lymphomas, among others. These secondary infections are separately treated using other medications as is conventional. During the period immediately following infection, persons with HIV seem to live on with little or no symptoms, but have persistent infections. Later in the disease, persons suffer mild immune system depression with various symptoms such as weight loss, malaise, fever, and swollen lymph nodes. The syndromes which are identified with such symptoms have been called persistent generalized lymphadenopathy syndrome (PGL) and AIDS related complex (ARC).

In all cases, those infected with the AIDS virus are believed to be persistently infective to others. Further, AIDS and AIDS related complex is, after some time, fatal.

A description of the mechanism by which the virus infects its host is given in an article by R. Yarchoan, and S. Broder, "Development of Antiretroviral Therapy for the Acquired Immunodeficiency Syndrome and Related Disorders", New England Journal of Medicine, 316: 557-564 (1987).

Considerable efforts are being directed toward the control of HIV by means of inhibition of the reverse transcriptase of HIV, required for replication of the virus. (V. Merluzzi et al., "Inhibition of the HIV-1 Replication by a Nonnucleoside Reverse Transcriptase Inhibitor", Science, 25: 1411 (1990)). For example, a currently used therapeutic compound, AZT (U.S. Patent No. 4,724,232) is an inhibitor of the viral reverse transcriptase. Unfortunately, many of the known compounds suffer from toxicity problems, lack of bioavailability, the development of viral resistance, short half-lives in vivo, or combinations thereof.

Therefore, this invention provides improvements in the pharmaceutical arsenal of compounds used for the treatment of medical indications which are initiated by uninhibited $PLA_2$, in addition to the use of such compounds for the prevention and treatment of infections in mammals by retroviruses. In particular, these improvements include novel A87689 compounds, their derivatives and pharmaceutically acceptable salts thereof, processes for producing such compounds, the above-mentioned multiple uses of such compounds, and therapeutic formulations which are of value in $PLA_2$ inhibition and management of retroviral infections in mammals.

Other objects, features and advantages will become apparent to those skilled in the art from the following description and claims.

The infrared (IR) absorption spectra of A87689 and exemplary salts and derivatives (in KBr pellet) are shown in Figures 1-5 as follows:

Figure 1 - A87689 calcium salt.
Figure 2 - A87689 free acid.
Figure 3 - A87689 tetraacetate calcium salt.
Figure 4 - A87689 tetramethyl ether calcium salt.
Figure 5 - A87689 penta-sodium salt.
Figure 6 is a computer-generated dendrogram constructed from fatty acid profiles.

This invention relates to a new fermentation product which has been designated "A87689". A87689, which is useful, inter alia, as an enzyme inhibitor, is generally purified from the culture medium in the form of an alkaline earth metal salt, such as the calcium salt. Further aspects of this invention are processes for producing A87689 by culturing a novel strain of the microorganism Amycolatopsis mediterranei, selected from NRRL 18815 and NRRL 18851, or an A87689-producing mutant thereof, and biologically purified cultures of the Amycolatopsis mediterranei strains which produce it. As those skilled in fermentation processes will recognize, although the compound is generally isolated from the microorganism in alkaline earth salt form, other forms of the compound may then be generated by derivatization in the laboratory, as described infra.

One should note that the name of the A87689-producing microorganisms was changed in 1986, when two new genera were split from the nocardioform actinomycetes. The new genus Amycolata and the genus Amycolatopsis were identified at that time by Lechevalier, et al. [M. P. Lechevalier, H. Prauser, D. P. Labeda, J. Ruan, "Two New Genera of Nocardioform Actinomycetes Amycolata New-Genus and Amycolatopsis New-Genus", Int. J. Syst. Bacteriol., 36: 29-37 (1986)].

In discussions of utility, the form of compound administered will be referred to, such as the "calcium salt of A87689" or the "free acid of A87689"; or if one or more than one of the derisetives is being discussed, it may be referred to as the "A87689 compounds."

The calcium salt of A87689 is a white, crystalline material which has the following characteristics:

Molecular weight: 1222.418
Empirical formula: $C_{66}H_{70}O_{20}Ca$

FAB-MS (M+1):
Found: 1223.4175
Calculated: 1223.4254

Solubility: Very slightly soluble in water (1 mg/mL) at pH 7; quite soluble in aqueous solution of pH 12.0 or greater; soluble in DMSO at 4 mg/mL; slightly soluble in methanol; insoluble in organic solvents such as EtOAc, acetone, diethyl ether, hydrocarbons, chloroform, higher alcohols.

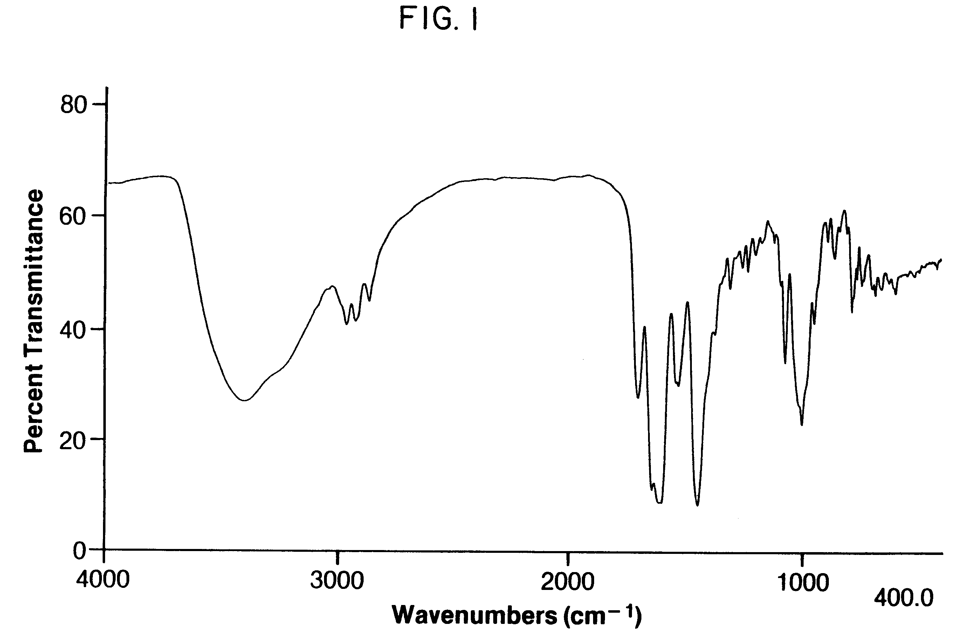

The IR spectrum of the calcium salt of A87689 (KBr pellet) is shown in Figure 1 of the accompanying drawings. The most significant absorption maxima occur at the following frequencies (cm$^{-1}$): 3402, 1711, 1609, 1534, 1449, 1078, 1006 and 792.

A87689 is capable of forming salts and has at least four hydroxyl groups which can be esterified or form ether derivatives. Thus, the $C_{1-6}$-alkanoyl ester and $C_{1-6}$-alkyl ether derivatives and the salts of A87689 and of these derivatives (A87689 compounds) are also part of this invention. The tetra-acetate and the tetramethyl ether derivatives, as free acids and salts, are examples. The A87689 compounds are useful as agents which reduce inflammation. The pharmaceutically-acceptable salts are particularly useful.

Base addition salts include those derived from inorganic bases, such as ammonium or alkali or alkaline earth metal hydroxides, carbonates, bicarbonates, and the like. Such bases useful in preparing the salts of this invention, thus, include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium, calcium and sodium salt forms are particularly preferred.

The term "$C_{1-6}$-alkanoyl" refers to straight or branched univalent aliphatic acyl groups of 1-6 carbon atoms including, for example, formyl, acetyl, propionyl, butyryl, $\alpha$-methylpropionyl, valeryl, $\alpha$-methylbutyryl, $\beta$-methylbutyryl, pivaloyl, and the like.

The term "$C_{1-6}$-alkyl" refers to straight or branched aliphatic chains of 1-6 carbon atoms including methyl, ethyl, propyl, isopropyl, n-butyl, isobutyl, sec.-butyl, n-pentyl, isopentyl, neopentyl, n-hexyl, isohexyl, 3-methylpentyl, 2,3-dimethylbutyl, and the like.

The terms "ester" and "ether" maintain their definitions as known in the art.

For purposes of discussion of taxonomy, the A. mediterranei cultures NRRL 18815 and NRRL 18851 will be called A87689 and A87689.1, respectively. Culture A87689 was isolated from a soil mixture (dilution). Derivative strain A87689.1 was produced by chemical mutagenesis. The novel compounds made by the A87689 and A87689.1 cultures will be called "A87689".

This invention also relates therefore, to a biologically purified culture of the microorganism Amycolatopsis mediterranei selected from NRRL 18815 or NRRL 18851, or an A87689-producing mutant thereof. These microorganisms are useful because they produce $PLA_2$ inhibitor and antiretroviral A87689 compounds.

Cultures A87689 and A87689.1 have been deposited in compliance with the Budapest Treaty, and made part of the stock culture collection of the Midwest Area Northern Regional Research Center, Agricultural Research Service, United States Department of Agriculture, 1815 North University Street, Peoria, Illinois, 61604, from which they have been assigned the following accession numbers: NRRL 18815 and NRRL 18851, respectively.

Taxonomic studies of cultures A87689 and A87689.1 were carried out by Frederick P. Mertz of Lilly Research Laboratories. Based on these studies, the microorganisms A87689 and A87689.1 are classified as new members of the species Amycolatopsis mediterranei. This classification is based on direct laboratory comparisons and examination of published descriptions of similar species.

## Methods Used

The taxonomic methods followed were those recommended by the International Streptomyces Project (ISP) for the characterization of Streptomyces species [Shirling, E. B. and Gottlieb, D., "Methods for characterization of Streptomyces species, " Int. J. Syst. Bacteriol. 16: 313-340, (1966)] and those recommended for the characterization of Nocardia species [Gordon, R. E., Barnett, D. A., Handerhan, J. E., and Pang, C. H., "Nocardia coeliaca, Norcardia autotrophica, and the Nocardin Strain," Int. J. Syst. Bacteriol. 24(1): 54-63 (1974).] ISCC-NBS Centroid color Charts, standard sample No.2106, were used to assign color names to the reverse side and to aerial hyphae (National Bureau of standards, 1958, U.S. Department of commerce, Washington, D.C.)

Morphology was studied using an optical light microscope and a scanning electron microscope (SEM). The isomer of diaminopimelic acid (DAP) and the carbohydrates in hydrolysates of whole cells were established by the chromatographic methods of Becker et al. [Becker, B.,Lechevalier, M. P., Gordon, R. E. and Lechevalier, H. E., "Rapid Differentiation between Nocardia and streptomyces by Paper Chromatography of Whole-cell Hydrolysates," Appl. Microbiol. 12,421-423 (1964)] and of Lechevalier and Lechevalier [Lechevalier, M. P. and Lechevalier, H., in A University Laboratory Approach, Dietz and Thayer (eds.), Society for Industrial Microbiology special Publication No. 6, Arlington, VA, pp. 227-233.]

Phospholipids were determined by the procedure of Lechevalier et al.[Lechevalier, M. P., stern, A. E. and Lechevalier, H. A., "Phospholipids in the taxonomy of Actinomycetes," in Actinomycetes, Schaal and Pulverer (eds.),Zbl. Bakt. suppl. 11 Gustav Fischer Verlag, (1981)].

Menaquinone composition was determined by following the procedures of R. M. Kroppenstedt [in Chemical Methods in Bacterial Systematics, M. Goodfellow and D. E. Minnikin (eds.), 1985, pp. 173-196] and M. D. Collins [ibid., pp. 267-285]. Fatty acid analysis was done using the HP 5898A Microbial Identification System of Miller and Berger [Miller, L. and Berger, T., "Bacterial Identification by Gas Chromatography of Whole Cell Fatty Acids," Hewlett-Packard Application Note 228-41, 8pp. (1985)].

Fatty acid methyl esters were made from lyophilized whole cells grown under identical conditions. Mycolic acids were determined by the methods proposed by Minnikin [Minnikin, D. E., Hutchinson, I. G., and Caldicott, A. B., "Thin-Layer Chromatography of Methanolysates of Mycolic Acid-Containing Bacteria, " J. Chromatography 188: 221-233(1980)].

## Cultural Characteristics

Culture A87689 grew well on complex and defined media. Culture A87689 did not produce aerial hyphae except for trace amounts of white aerial hyphae on ISP medium 4. The reverse side had a distinctive orange pigmentation. On ISP medium 2 produced a faint light brown soluble pigment which was released into the medium. Table 1 summarizes these cultural characteristics.

## Table 1. Cultural characteristics of A87689[1]

| Medium | Growth | Reverse Color | Aerial Growth | Soluble Pigment |
|---|---|---|---|---|
| ISP medium 2 | Abundant | 50. s.O | None | L. Brown |
| ISP medium 3 | None | | | None |
| ISP medium 4[2] | Abundant | 50. s.O | Trace | None |
| ISP medium 5 | None | | | None |
| ISP medium 7 | Poor | 90. gy.Y | None | None |
| ATCC No. 172 | Good | 50. s.O | None | None |
| BENNETTS | Fair | 53. m.O | None | None |
| Calcium-malate | Abundant | 48. V.O | None | None |
| CZAPEKS | Abundant | 50. s.O | None | None |
| TPO[3] | Abundant | 71. m.OY | None | None |

[1]Incubated at 30°C for 28 days

[2]Mycelium color 263. White

[3]Tomato Paste Oatmeal Agar

## Morphological Characteristics

Culture A87689 exhibited no fragmentation. The hyphae segmented into long strands of conidia arranged in typical cobweb morphology, characteristic of nonstreptomycete actinomycetes. The conidia are cylindrical in shape, have a smooth surface, and average 1.4 X 0.4 mm. Sporangia or motile cells are not present.

## Physiological Characteristics

Culture A87689 produced acid from the following carbohydrates: adonitol, D-arabinose, L-arabinose, cellobiose, D-galactose, D-fructose, D-glucose, glycerol, glycogen, i-inositol, inulin, lactose, D-maltose, mannitol, D-mannose, melebiose, raffinose, L-rhamnose, D-ribose, sucrose, trehalose, and D-xylose.

A87689 did not produce acid from cellulose, dextrin, dulcitol, ethanol, i-erythritol, D-melezitose, methyl-D-glucoside, salicin, sorbitol, L-sorbose and xylitol.

Culture A87689 utilized the following carbohydrates with ISP medium 9 as the basal medium: L-arabinose, D-fructose, D-galactose, D-glucose, inositol, D-mannitol, D-raffinose, L-rhamnose, salicin, sucrose, and D-xylose. Culture A87689 utilized sodium salts of the following organic acids (as sole sources of carbon for energy and growth): acetate, butyrate, citrate, formate, lactate, malate, mucate, oxalate, propionate, pyruvate, and succinate. Culture A87689 was unable to utilize benzoate and tartrate. A87689 decomposed casein, hypoxanthine and L-tyrosine. It was unable to hydrolyse adenine and xanthine. A87689 produced esculinase, gelatinase, $H_2S$, phosphatase and urease. It did not produce melanoid pigments. A87689 grew between 15°C and 42°C. A87689 tolerated up to 4% NaCl. The culture is sensitive to lysozyme.

## Cell-Wall Analysis

Hydrolyzed whole cells contained mesodiaminopimelic acid (DAP). Diagnostic sugars in the whole cell extracts were arabinose and galactose. Phospholipids present were phosphatidyl ethanolamine (PE), phosphatidyl inositol (PI), and diphosphatidyl glycerol (DPG). A87689 has a type IV cell wall composition and a type A whole-cell sugar pattern [Lechevalier, M. P. and Lechevalier, H., "Chemical composition as a criterion in the classification of aerobic actinomycetes," Int. J. Syst. Bacteriol. 20: 435-443] and a type PII phospholipid pattern [Lechevalier, M. P., Stern, A. E., Lechevalier, H. A., "Phospholipids in the Taxonomy of Actinomycetes," in Actinomycetes, Schaal and Pulverer (eds.) Zbl. Bakt. Suppl. II Gustav Fischer Verlag, Stuttgart, New York]. Mycolic acids were not present in whole cell hydrolysates. The major menaquinones detected were MK-9 ($H_4$), plus minor amounts of MK- 8($H_4$), MK- 9 ($H_6$), and MK-10 ($H_4$).

## Identity of A87689

Culture A87689 is a non-streptomycete strain with a type IV cell wall, type A whole cell sugar pattern, type PII phospholipid pattern, and no mycolates. These chemotaxonomic characteristics plus its cultural and morphological properties place A87689 in the genus Amycolatopsis. There are six species and one subspecies in this genus: A. azurea, A. fastidiosa, A. mediterranei, A. orientalis, A. orientalis subsp. lurida, A. rugosa, and A. sulphurea. When compared to these existing strains, culture A87689 was most similar to A. mediterranei. Culturally, it was almost identical. The morphological characteristics were minimal due to the absence of aerial hyphae, but its primary growth was identical to A. mediterranei. The biochemical characteristics of A87689 and A. mediterranei are also almost identical. Table 2 reports the differences between A87689 and A. mediterranei.

## Table 2. Differential characteristics of A87689 and *Amycolatopsis mediterrannei.*

| Characteristic | A87689 | *A. mediterranei* |
|---|---|---|
| Decomposition of: | | |
| L-Tyrosine | + | - |
| Resistant to: | | |
| 4% NaCl | + | - |
| Growth at: | | |
| 42°C | + | - |
| Acid produced from: | | |
| Adonitol | + | - |
| Inulin | + | - |

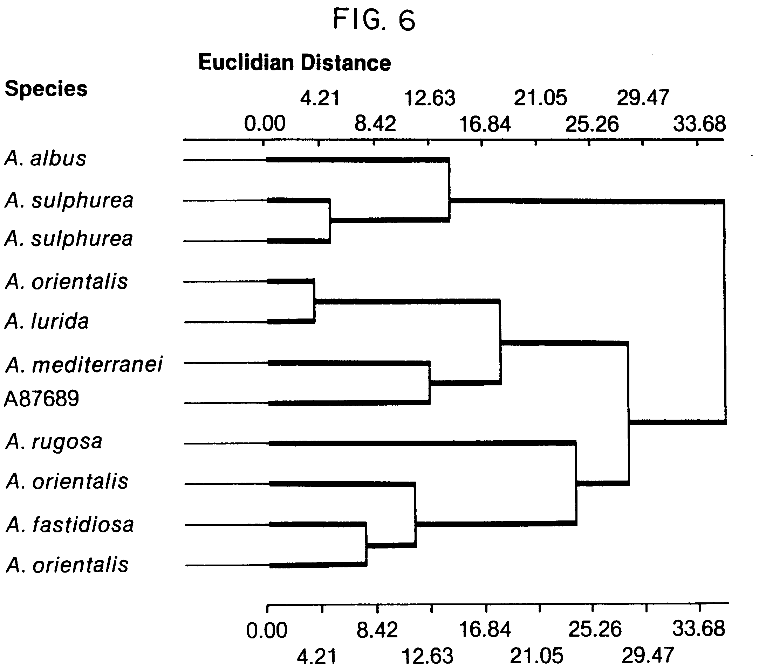

Fatty acid analysis was done on A87689 and all the available Amycolatopsis strains. Table 3 presents a comparison of the percentage fatty acids. A computer-generated dendrogram constructed from fatty acid profiles is presented in Figure 6. This dendrogram shows the relationship of A87689 to the other Amycolatopsis species measured by Euclidean distance. Cultures showing relatedness below the level of 10.00 are interpreted as being synonymous, that is, the same species. A87689 is related to A. mediterranei at the level of 12.00.

Principal component analysis is a branch of multivariate statistics that deals with internal relationships of a set of variables. In this analysis the greatest amount of variance within the original data or test results is expressed as principal components [Alderson, G. "The application and relevance of non hierarchic methods in bacterial taxonomy," in Computer-assisted bacterial systematics, Goodfellow, M., Jones, D., and Priest, F. G. (eds.) Academic Press, New York (1985)]. Thus, a plot showing scatter or variability can be constructed. Relationships can then be evaluated by examining this variance, and a microbial population can be characterized. A two-dimensional principal component plot based on fatty acids was constructed and showed culture A87689 clustering with A. mediterranei. Although there is variation between A87689 and A. mediterranei, this variation is a strain and not a species difference. Therefore, culture A87689 is classified as a strain of Amycolatopsis mediterranei. [Lechevalier, M. P., Hauser, H., Labeda, D. P., Ruan, J.S., "Two new genera of nocardioform actionomycetes: Amycolata gen. nov. and Amycolatopsis gen. nov., " Int. J .Syst. Bacteriol., 36: 21-37.

Table 3. Percentage fatty acid composition of A87689 and Amycolatopsis species.

| Fatty Acid | A87689 | A. orientalis | A. lurida | A. fastidiosa | A. rugosa | A. mediterranei | A. sulphurea |
|---|---|---|---|---|---|---|---|
| 14:0 Iso | 2.29 | 6.36 | 5.68 | 5.56 | 0.98 | 2.35 | 2.64 |
| 15:0 Iso | 7.90 | 9.98 | 9.32 | 10.76 | 2.23 | 15.51 | 17.66 |
| 15:0 Anteiso | 1.03 | 1.69 | 1.53 | 0.70 | - | 2.36 | 6.14 |
| 15:1 B$^1$ | 2.09 | - | - | 3.12 | 4.44 | 1.89 | - |
| 15:0 | 4.58 | 6.25 | 6.37 | 2.82 | 0.54 | 4.65 | 5.51 |
| 16:1 Iso H | 5.19 | 0.45 | 1.00 | 12.76 | 31.39 | 4.21 | - |
| 16:0 Iso | 37.10 | 21.46 | 24.20 | 39.71 | 29.80 | 29.32 | 13.24 |
| 16:0 Anteiso | 0.21 | - | - | 0.23 | - | 1.23 | 0.53 |
| 15:0 Iso2OH | - | 7.64 | 6.50 | - | - | - | 17.25 |
| 16:1 Cis 9 | 2.74 | - | - | 0.82 | 4.55 | 1.18 | - |
| 16:0 | 0.93 | 1.58 | 1.10 | 0.37 | 0.27 | 0.65 | 8.29 |
| 17:1 Iso G | 1.55 | 0.32 | 0.56 | - | - | 1.76 | - |
| 17:0 Iso | 0.65 | 0.57 | 0.51 | 1.02 | 0.68 | 1.02 | 2.74 |
| 17:0 Anteiso | 1.45 | 0.88 | 0.85 | 1.05 | 0.57 | 4.29 | 6.71 |
| 17:1 B | 15.65 | 18.75 | 18.61 | 1.89 | 3.32 | 12.37 | 1.70 |
| 17:1 C | 7.19 | 12.99 | 13.50 | 15.64 | 19.19 | 4.28 | 7.23 |
| 17:0 | 1.79 | 8.56 | 6.84 | 0.72 | 0.17 | 3.02 | 8.92 |
| 18:1 Iso F | 5.95 | 1.15 | 2.45 | - | 0.20 | 8.15 | - |

$^1$B, C, F, G, and H, indicate double bond positions/configurations that are unknown.

## Comparison of Derivative Strains

The A87689.1 strain is sufficiently similar macroscopically to the A87689 strain to be classified as a strain of Amycolatopsis mediterranei. The two strains differ in the amount of A87689 they produce. The A87689.1 strain produces approximately two-fold more A87689 compound than the A87689 strain produces.

A87689 is produced by culturing an A87689-producing strain of Amycolatopsis mediterranei under submerged aerobic conditions in a suitable culture medium until a recoverable amount of A87689 is produced. A87689 can be recovered using various isolation and purification procedures understood in the art.

The culture medium used to grow the Amycolatopsis mediterranei cultures can be any one of a number of media. For economy in production, optimal yield, and ease of product isolation, however, certain culture media are preferred. Thus, for example, preferred carbon sources in large-scale fermentation are glucose and maltose, although ribose, xylose, fructose, galactose, mannose, mannitol, soluble starch, potato dextrin, methyl oleate, oils such as soybean oil and the like can also be used.

Preferred nitrogen sources are cottonseed flour, peptionized milk and digested soybean meal, although fish meal, corn steep liquor, yeast extract, enzyme-hydrolyzed casein, beef extract, and the like can also be used.

Among the nutrient inorganic salts which can be incorporated in the culture media are the customary soluble salts capable of yielding zinc, sodium, magnesium, calcium, ammonium, chloride, carbonate, sulfate, nitrate and like ions.

Essential trace elements necessary for the growth and development of the organism should also be included in the culture medium. Such trace elements commonly occur as impurities in other substituents of the medium in amounts sufficient to meet the growth requirements of the organism.

For preferred production of substantial quantities of A87689, submerged aerobic fermentation in stirred bioreactors is preferred. Small quantities of A87689 may be obtained by shake-flask culture. Because of the time-lag in production commonly associated with inoculation of large bioreactors with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger bioreactor. The vegetative inoculum medium can be the same as that used for larger fermentations, but other media are also suitable.

A87689 compound is produced by the A87689-producing organisms when grown at temperatures between about 27° and about 32°C. Optimum temperatures for A87689 production appear to be about 30°C.

As is customary in submerged aerobic culture processes, sterile air is blood into the vessel from the bottom while the medium is stirred with conventional turbine impellors. In general, the aeration rate and agitation rate should be sufficient to maintain a level of dissolved oxygen of at least 45% of air saturation with an internal vessel pressure of 5 atmospheres.

Production of the A87689 compound can be followed during the fermentation by testing extracts of the broth for $PLA_2$ activity. The snake venom (Naja naja) $PLA_2$ assay system described infra is a useful assay for this purpose.

Following its production under submerged aerobic fermentation conditions, the A87689 compound can be recovered from the fermentation medium by methods used in the art. The A87689 produced during fermentation of the A87689-producing organism occurs mainly in the broth.

A87689 can be recovered from the biomass by a variety of techniques. A preferred technique involves filtering whole fermentation broth with a filter aid such as diatomaceous earth. The filtrate thus obtained is then adsorbed onto a high porous polymer adsorbent of the polystyrene type, such as DIAION HP-20 (Mitsubishi Chemical Industries, Ltd., Tokyo) or Amberlite XAD-2 or AMBERLITE XAD-4 (Rohm and Haas, Philadelphia, PA). The adsorbent is washed with water and eluted with an organic-aqueous solution, such as methanol-water. The eluate is lyophilized and concentrated. In a preferred separation procedure, the lyophilized material is dissolved in dimethyl sulfoxide (DMSO) and chromatographed on a reverse-phase silica gel adsorbent ($C_8$ or $C_{18}$). Fractions are eluted in organic-aqueous buffer, such as acetonitrile:ammonium acetate.

## Phospholipase A2 Inhibitory Activity

The compounds of this invention are useful for the reduction of inflammation of tissues because they inhibit $PLA_2$, an enzyme that is involved in the regulation of tissue inflammation.

The A87689 compounds have shown inhibitory activity in a number of assays which specifically detect $PLA_2$ activity. More specifically, the compounds were active in an in vitro assay utilizing isolated snake venom (Naja naja) $PLA_2$, an in vitro assay utilizing purified human cytosolic $PLA_2$, and a whole cell in vitro assay which detects the inhibition of secretion of metabolites of phospholipids (leukotriene and thromboxane).

Significantly, the claimed compounds were not active when tested in a 5-lipoxygenase assay, revealing their specificity for $PLA_2$.

In addition, the compounds have shown activity in in vivo assays for anti-inflammatory activity. Specifically, the compounds were active in three adjuvant-induced arthritis models.

Two salt forms of A87689 were tested in the biological assays- One form was soluble in both water and DMSO. This is the sodium salt of A87689. The other form was insoluble in water, but soluble in DMSO. This is the calcium salt of A87689. The latter form was tested either as a solution in DMSO or as a suspension in 1% carboxymethyl-cellulose (CMC).

In addition, the activity of derivatives and salts of A87689 were compared in one of the biological assays, the snake venom (Naja naja) PLA$_2$ assay.

Thus, the present invention is also directed to a method for reducing inflammation in animals which comprises administering to an animal an inflammation-inhibiting amount of an A87689 compound.

One preferred embodiment of the invention is a method for reducing the swelling which occurs in arthritic animals, which comprises administering to the animal an amount of A87689 compound sufficient to reduce the swelling.

In another preferred embodiment, the invention is directed to a method for treating asthma, which comprises administering to an animal an amount of A87689 compound sufficient to reduce the inflammation of the respiratory system that occurs in asthmatic animals.

In yet another embodiment, this invention provides a method for treating psoriasis, which comprises administering to an animal suffering from psoriasis an amount of A87689 compound that is effective to reduce the psoriatic lesions.

## Retroviral Activity

Once produced and isolated, A87689 compounds, when administered to mammals, are useful for the prevention of retroviral infection in such mammals, are useful for treatment of such mammals infected by retroviruses, or both.

Because A87689 compounds possess this activity against retroviral infections, the term "managing" or "management" of retroviral infections, as used throughout this specification, includes the prevention of an initial or recurring infection by one or more retroviruses, and also includes the treatment of mammals following an initial or recurring retroviral infection.

A further embodiment of the present invention includes a method of managing retroviruses of the subfamily oncovirinae in mammals, especially human T-cell leukemia virus, comprising administering to said mammal an oncoviral-managing amount of a compound selected from the A87689 compounds or a pharmaceutically acceptable salt thereof.

An especially preferred embodiment of this invention includes a method of managing HIV in human beings comprising administering to said human being an HIV-managing amount of a compound selected from the A87689 compounds or a pharmaceutically acceptable salt thereof.

A87689 compounds will also manage the AIDS retroviruses. Thus, another especially preferred embodiment of this invention includes a method of managing AIDS in human beings comprising administering to said human being an AIDS-managing amount of a compound selected from the A87689 compounds or a pharmaceutically acceptable salt thereof.

Despite the recognized therapeutic potential associated with presently known anti-HIV and anti-AIDS agents, and the enormous reSearch effort thus far, a single, viable, therapeutic agent has not yet emerged. Therefore, a further especially preferred embodiment of this invention includes a method of managing HIV and AIDS in human beings comprising administering to said human being an HIV-managing or an AIDS-managing amount of a first component which is selected from an A87689 compound or a pharmaceutically acceptable salt thereof, and an effective amount of a second component which is one or more HIV- and/or AIDS-inhibiting compounds or a pharmaceutically acceptable salt thereof.

Particularly useful second component compounds include nucleoside reverse transcriptase inhibitors such as 3'-azido-2',3'-dideoxythymidine (AZT; U.S. Pat. No. 4,724,232); and 2',3'-dideoxycytidine (DDC) and 2',3'-dideoxyinosine (DDI; Mitsuya, H., et al., Proc. Natl. Acad. Sci., 83: 1911-1915 (1986)), and the like. Other useful second component compounds include the non-nucleoside derivatives of tetrahydroimidazo[4,5,1-jk] [1,4]benzodiazepin-2 (1H)-one and -thione, and the like (TIBO derivatives; Debyser, et al., Proc. Natl. Acad. Sci., 88: 1451-1455 (1991)). However, TIBO derivatives are only active against HIV-1 replication. Further useful second component compounds include HIV protease inhibitors. For example, EPA 361 341; EPA 346 847; EPA 402 646; and EPA 337 714 all disclose compounds which are said to be useful as HIV protease inhibitors. First component compounds, which are different than the A87689 compound administered as a first component compound, may also be used as second component compounds.

In an additional preferred embodiment, the invention is directed to a pharmaceutical formulation comprising an effective amount of a first component, as described above, and a second component, also described above, together with a pharmaceutically acceptable carrier, excipient or diluent.

As used above and throughout this specification, the term "effective amount" means that dosage or active compound(s) sufficient to provide therapeutic treatment of the specified medical indication.

The term "active compound", as used throughout this specification, refers to at least one compound selected from A87689, its derivatives, or pharmaceutically acceptable salts thereof, and anti-HIV and/or anti-AIDS agents, or the pharmaceutically acceptable salts thereof.

For therapeutic treatment of the specified indications, first component compounds, with or without a second component compound, may be administered as such, or can be compounded and formulated into pharmaceutical compositions in unit dosage form for parenteral, transdermal, rectal, nasal or intraveneous administration or, preferably, oral administration. Such pharmaceutical compositions are prepared in a manner well known in the art and comprise at least one active compound selected from the A87689 compounds optionally including one or more second component compounds associated with a pharmaceutically carrier. In such a composition, the active compound and, if included, the second component compound(s), are known as active ingredients. In making the compositions, the active ingredient(s) will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient. Thus, the composition can be in the form of tablets, pills, powders, lozenges, sachets, cachets, elixirs, emulsions, solutions, syrups, suspensions, soft and hard gelatin capsules, sterile injectable solutions, and sterile packaged powders.

Some examples of suitable carriers, excipients, and diluents include lactose, dextrose, sucrose, sorbitol, mannitol, starches, gum acacia, calcium phosphate alginates, calcium salicate, microcrystalline cellulose, polyvinylpyrrolidone, cellulose, tragacanth, gelatin, syrup, methyl cellulose, methyland propylhydroxybenzoates, talc, magnesium stearate, water, and mineral oil. The formulations can additionally include lubricating agents, wetting agents, emulsifying and suspending agents, preserving agents, sweetening agents or flavoring agents. The compositions may be formulated so as to provide quick, sustained, or delayed release of the active ingredient(s) after administration to the patient by employing procedures well known in the art. For oral administration, a compound, optionally including a second component compound, can be admixed with carriers and diluents molded into tablets or enclosed in gelatin capsules. The mixtures can alternatively be dissolved in liquids such as 10% aqueous glucose solution, isotonic saline, sterile water, or the like, and administered intravenously or by injection.

The compositions are preferably formulated in a unit dosage form, each dosage containing from about 1 to about 500 mg and, more frequently, from about 5 to about 300 mg of the active ingredient(s). The term "unit dosage form" refers to physically discreet units suitable as unitary dosages for human subjects and other mammals, each unit containing a predetermined quantity of active material calculated to produce the desired therapeutic effect, in association with the required pharmaceutically acceptable carrier. Such compositions may contain an A87689 compound as an active ingredient or may contain an A87689 compound plus a second component compound as active ingredients. By "pharmaceutically acceptable", it is meant the carrier, diluent or excipient must be acceptable with the other ingredients of the formulation and not deleterious to the recipient thereof.

The following formulation examples are only illustrative and are not intended to limit the scope of the invention in any way. "The meaning of the term active ingredient" is as defined above.

Formulation 1

Hard gelatin capsules are prepared using the following ingredients:

|  | Quantity (mg/capsule) |
| --- | --- |
| Active ingredient(s) | 250 |
| Starch, dried | 200 |
| Magnesium stearate | 10 |
| Total | 460 mg |

Formulation 2

A tablet is prepared using the ingredients below:

|                              | Quantity (mg/capsule) |
| ---------------------------- | --------------------- |
| Active ingredient(s)         | 250                   |
| Cellulose, microcrystalline  | 400                   |
| Silicon dioxide, fumed       | 10                    |
| Stearic acid                 | 5                     |
| Total                        | 665 mg                |

The components are blended and compressed to form tablets each weighing 665 mg

Formulation 3

An aerosol solution is prepared containing the following components:

|                              | Weight |
| ---------------------------- | ------ |
| Active ingredient(s)         | 0.25   |
| Ethanol                      | 25.75  |
| Propellant 22 (Chlorodifluoromethane) | 70.00 |
| Total                        | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

Formulation 4

Tablets, each containing 60 mg of active ingredient, are made as follows:

|                                          |         |
| ---------------------------------------- | ------- |
| Active ingredient(s)                     | 60 mg   |
| Starch                                   | 45 mg   |
| Microcrystalline cellulose               | 35 mg   |
| Polyvinylpyrrolidone (as 10% solution in water) | 4 mg |
| Sodium carboxymethyl starch              | 4.5mg   |
| Magnesium stearate                       | 0.5 mg  |
| Talc                                     | 1 mg    |
| Total                                    | 150 mg  |

The active ingredient, starch and cellulose are passed through a No. 45 mesh U.S. sieve and mixed thoroughly. The aqueous solution containing polyvinyl- pyrrolidone is mixed with the resultant powder, and the mixture then is passed through a No. 14 mesh U.S. sieve. The granules so produced are dried at 50°C and passed through a No. 18 mesh U.S. Sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| Active ingredient(s) | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| Active ingredient(s) | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions, each containing 50 mg of active ingredient per 5 ml dose, are made as follows:

| | |
|---|---|
| Active ingredient(s) | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| Active ingredient(s) | 100 mg |
| Isotonic saline | 1,000 ml |

The A87689 compounds are effective over a wide dosage range. For example, daily dosages will normally fall within the range of about 0.1 mg/kg to about 50 mg/kg of body weight. In the treatment of adult humans, the dosage range from about 5 mg/kg to about 25 mg/kg, in single or divided doses, is preferred. However, it will be understood that the amount of the compound actually administered will be determined by a physician in light of the relevant circumstances including the relative severity of a disease state, the choice of compound or compounds to be administered, the age, weight, and response of the individual patient, and the chosen route of administration. Therefore, the above dosage ranges are not intended to limit the scope of this invention in any way. Dosage ranges for second component compounds are known in the art and should be used accordingly.

## A87689 in In Vitro Phospholipase A2 Models In vitro snake venom (Naja naja) assay

In this assay, endogenous phospholipases produced by the microorganisms are removed from the broth by filtration through a membrane filter (mw cut-off of 10,000, Centricon). Broth (100 mL) is incubated in a reaction mixture of a total volume 0.540 mL for one hour at 37°C. The reaction mixture consists of 0.24 mM 1-palmitoyl-2-[6-[(7-nitro -2-1,3-benzoxadiazol-4-yl)amino]caproyl] phosphatidylcholine (PLA$_2$ chromogenic substrate, Avanti, Polar-Lipids, Inc., Alabaster, AL), 0.76 mM phosphatidyl choline, 10 mM TRITON X-100 (surfactant, Sigma), 10 mM CaCl$_2$, 37 mM TRIS-HCl at pH 8.5 (Sigma), 0.07 units/mL Naja naja PLA$_2$ (Sigma). The reaction is stopped by adding 0.2 mL of 0.25 M ethylene diamine tetraacetate (EDTA). The product, 7-nitro-2-1,3-benzoxadiazol-4-yl]amino]caproic acid, is separated from the substrate by high performance liquid chromatography (HPLC). The assay mixture (20 mL) is injected onto a C$_8$ column (0.46 X 15 cm, ZORBAX) with a C$_8$ guard cartridge [Solvent, acetonitrile: water: acetic acid (40:60:1); Flow rate: 1.5 mL/min.; Detection of product: UV at 453 nm. Retention time: 3.72-3.76 minutes]. Tables 4 and 5 show the effect of A87689 compounds in this assay.

## Human cytosolic PLA$_2$assay

Cytosolic PLA$_2$ purified from human monoblast U937 cells was used to test the inhibitory activity of A87689 in vitro. The assay was performed as described in Kramer et al. [Kramer, R. M., Roberts, E. F. Manetta, J., and Putnam, J. E., "The Ca$^{++}$ -sensitive Cytosolic Phospholipase A$_2$ Is a 100-kD Protein in Human Monoblast U937 Cells," J. Biol. Chem. 266(8): 5268-5272]. Briefly, 50 mL of sonicated liposomes containing 1-palmitoyl-2-[$^{14}$C] arachidonoyl-sn-glycero-3-phosphocholine and 1,2-dioleoyl glycerol (2:1 molar ratio) are added to varying micromolar concentrations of test compound in 150 mL of assay buffer. This assay mixture contains 1 mM CaCl$_2$, 2 mM 2-mercaptoethanol, 150 mM NaCl, 50 mM N-[2-hydroxyethyl]piperazine-N$^1$-[2-ethane sulfonic acid] (HEPES) pH 7.5 and 1 mg/mL bovine serum albumin (BSA). To each tube, the enzyme (5 mL, diluted to yield 10-20% hydrolysis of the substrate) is added and the mixture is incubated at 37°C for 15 min. The reaction is stopped with Doles reagent [Zhang, Y. Y., Deems, R. A., and Dennis, E. A. in Methods in Enzymology, vol. 197, P. 457, Academic Press, New York (1991)]. The free arachidonic label is extracted with heptane:water (1.2:1). The upper phase is removed to tubes containing 0.1 g silica gel, mixed, centrifuged at 1500xg and the supernatant is removed for scintillation counting. The degree of inhibition is calculated and graphed for each drug concentration, and expressed as the amount that inhibits the maximum reaction by 50% (IC$_{50}$.) The effect of A87689 in this assay is presented in Table 4. In this assay, the A87689 compound were much more potent than were the classic inhibitors of venom or pancreatic PLA$_2$s, such as para-bromophenacyl bromide, mepacrine or manoalide.

## Whole cell PLA$_2$Test

Human promyelocytic leukemia cells (HL-60), induced to differentiate into granulocyte-like cells by growth in the Presence of 1.5% DMSO, release the leukotriene B$_4$ (LTB$_4$) and the thromboxane B$_2$ (TXB$_2$) in response to stimulation with the calcium ionophore A23187. The release of those eicosanoids is inhibited by treatment of the cells with the PLA$_2$ inhibitor manoalogue ([E, E]-2-[3-(2,5-dihydro-2-hydroxy-5-oxo-3-furanyl)propylidene]- 6,10-dimethyl-5,9-undecadienal). Manoalogue is an inhibitor of a secreted form of PLA$_2$, (Reynolds, et al.) and an intracellular form of PLA$_2$ (Dennis, et al.). [Reynolds, et al. J. American Chemical Society 110: 5172, (1988); Dennis, et al. J. Biol. Chem. 264: 8520, (1989).]

The protocol for the HL-60 cell assay is as follows: HL-60 cells obtained from American Type Culture Collection (ATCC) are grown in suspension culture in RPMI 1640 medium (Gibco) supplemented with 10% fetal bovine serum, in a humidified atmosphere of 5% CO$_2$ at 37°C. Cells are induced to differentiate into granulocyte-like cells by culture in the presence of 1.5% DMSO for 7 days. Cells are harvested by centrifugation at 4°C for 5 minutes and washed once in 118 mM NaCl, 4.6 mM KCl, 24.9 mM NaHCO$_3$, 1 mM KH$_2$PO$_4$ and 11.1 mM D-glucose, 5 mM HEPES, pH 7.4, supplemented with 0.1% BSA, 1.1 mM MgCl$_2$ and 1 mM CaCl$_2$. Washed cells are resuspended to a concentration of 1x10$^6$ cells/mL and prewarmed to 37°C for 5 minutes prior to initiation of the assay. Cells are preincubated for 5 minutes with inhibitors, followed by a 5 minute incubation with a calcium ionophore, A23187. Reactions are terminated by centrifugation. Supernatants are decanted and processed for the enzyme immunoassays (EIAs). Enzyme immunoassays for leukotriene $B_4$ and thromboxane $B_2$ are performed in a final volume of 150 mL containing 100 mM potassium phosphate buffer, pH 7.4, 0.4 NaCl, 1 mM EDTA, 1.5 mM $NaN_3$ and 0.1% BSA with 5 mL sample for $LTB_4$ and 50 mL for $TXB_2$. Samples are then incubated with antibody and tracer in 96 well microtiter plates (which are precoated with 5 mg mouse anti-rabbit IgG antibody per well) at room temperature overnight. After washing off excess antibody and tracer, 200 mL of Ellman's reagent (Immunoassay kit, Cayman Chemical Company) are added to the well, and samples are incubated at 25°C for 4 hours. OD at 405 nm is measured. Inhibitors, the calcium ionophore A23187, and test compounds are dissolved in DMSO as stock solutions (10mM) and added to the assays at serious concentrations in a total volume of 10 mL. The effect of A87689 in this assay is reported in Table 4.

## Table 4. Activity of A87689 in PLA₂ In Vitro Assays

| | $IC_{50}$ | |
|---|---|---|
| Assay | Calcium salt | Free Acid. |
| Snake venom (Naja naja) | | |
| PLA₂ | 8.0 mg/mL | 6.3 mg/mL |
| Purified human | | |
| cytosolic PLA₂ | 22 mg/mL | 17.2 mg/mL |
| Whole cell HL-60 | | |
| cell assay | inactive | * |

\* The free acid has some activity at 10 mM, but inhibitory activity plateaus at 30-40% inhibition.

## Table 5. Activity of A87689 Compounds in the Snake Venom (Naja naja) PLA$_2$ Assay

| Compound | IC$_{50}$ |
| --- | --- |
| Free Acid | 6.3 mg/mL |
| Tetra-acetate Calcium Salt | 9.5 mg/mL |
| Penta-Sodium Salt | 6.7 mg/mL |

### A87689 in In Vivo Arthritis Models

A87689 was tested in three different in vivo arthritis models designed to test for antiinflammatory activity. In each of these animal models, the hind paw of a rat is injected with a substance which induces an arthriticlike state in the paw joints which is evidenced by the presence of swollen, inflamed soft tissue and bone damage. This state is a form of induced arthritis so that agents which have antiinflammatory activities can be identified through the reduction in inflammation of the tissue and reduction in bone damage.

Test compound was administered orally and by intraperitoneal and subcutaneous injection. Preferably, oral administration is used; however, parenteral dosing of A87689 resulted in excellent antiinflammatory activity. Intraperitoneal injection of many irritant materials often results in peritonitis. Such peritonitis is correlated with antiinflammatory activity as evidenced by reduction in soft-tissue inflammation. In addition, subcutaneous injection of irritant materials may also result in nonspecific antiinflammatory activity. Each experiment was run in parallel with nonspecific irritant controls, such as 1% carrageenin or dilute acetic acid, in order to note how much antiinflammatory activity was nonspecific.

### Lipoidalamine-Induced Arthritis Model

The lipoidalamine-induced arthritis test is a model of systemic inflammation and arthritis induced by the adjuvant substance N, N-dioctadecyl-N',N'-bis(2-hydroxyethyl)propanediamine (LA) suspended in mineral oil. The disease that results is very similar to that occurring in Freund's complete adjuvant (FCA) treated rats according to Chang [Chang, Y., Pearson, C. M., and Abe, C., "Adjuvant polyarthritis IV. Induction by a synthetic adjuvant: immunologic, histopathologic, and other studies," Arth. Rheum.23: 62-71, (1980)]. Inflammation occurs in many tissues but is most striking in the spleen and joints. Compounds are tested for their ability to modulate splenic and joint inflammation in established disease (treatment starts on day 9).

The animals (Lewis/Sprague Dawley, males, 200-220 grams) are divided into groups of 5 and housed in hanging cages with food and water ad libitum. On day 0 they are injected with 7.5 mg of LA subcutaneously at the base of the tail. On day 9 the animals are weighed and compound administration is started. The compounds are suspended, using a tissue homogenizer, in a 1% CMC solution. The screening dose is 50 mg/kg orally. Compounds are administered once daily for 5 days (days 9-13) with no dosing on day 14. On day 15 the animals are weighed again and hind paw volume measured using a water manometer. The animals are then anesthetized and bled via cardiac puncture. After the animals are euthanized, the spleen is removed and weighed and the hind feet are removed just above the ankle joint and weighed. The individual blood samples are analyzed for fibrinogen. Means and standard errors for all of these parameters are then calculated, and percent differences from the diseased (LA) control are figured. Statistical significance is determined utilizing the Dunnet T calculation on the raw data. The positive control for the LA arthritis test is dexamethasone. Dexamethasone at a dose of 0.1 mg/kg orally will inhibit paw swelling by 80-90% and spleen weight by 90-100%. Effects on fibrinogen are more variable, but it is usually inhibited by 50-70%.

Compounds that show statistical inhibition of paw swelling or spleen weight are rerun at 50 mg/kg; if activity is confirmed, a dose response is run. Compounds may also be tested in the two complete Freund's adjuvant-induced arthritis models in rats discussed infra.

When calcium salt of A87689 was administered subcutaneously at a dosage of 14 mg/kg in DMSO in this test, the swelling of the injected paw was inhibited by 46%.

When the sodium salt of A87689 was solubilized in water and administered subcutaneously at a dosage of 20 mg/kg in this test, it inhibited swelling of paw volume by 30%. Likewise, when the sodium salt A87689 was solubilized in DMSO and administered intraperitoneally at 10 mg/kg, it inhibited paw swelling by 15%. However, when the sodium salt was solubilized in DMSO and administered subcutaneously at 10 mg/kg, it inhibited paw swelling by 65%.

## Freund's Complete Adjuvant Induced Polyarthritis

Two of the in vivo models used were based on the subplantar introduction of FCA into the hind paw of rats, and measurement of the resulting inflammation. The A87689 compound was administered by three methods: intraperitoneally (IP), orally, (PO) and subcutaneously (SC). The two models, which are based on the introduction of FCA into the paw of rats, are the "developing test" and the "established test".

In the developing test, male Wistar-Lewis rats weighing 220-240 grams are used for testing (Harlan Sprague Dawley Laboratories)- The animals are acclimated to laboratory conditions for seven days prior to use. Animals are provided Purina Lab Chow and water ad libitum, and lights are cycled 12 hours on and off. FCA is prepared by diluting PERRIGEN (adjuvant, Calbiochem) 1:6 with mineral oil, resulting in a final concentration of 0.083%. On day 0 the rats are given a single subplantar injection of 0.1 mL of FCA suspension in the right hind paw. Animals are divided into groups of 5 and weighed. In the developing test, treatment with the test compound is started on day 0. Normal and FCA controls are administered by placebo vehicle. Paw volumes are measured by an electrically amplified aqueous displacement instrument three days a week. The animals are weighed again on day 14, and the treatment dosage is appropriately adjusted. The test is terminated on day 28, at which time the animals are again weighed, paw volumes are measured, and radiographs are made of both hind paws. Injected and uninjected paw volume curves are made, and areas under the curve are calculated. These areas are compared to the FCA control, and a percent inhibition from the FCA control is calculated. Bone damage scores are determined from analysis of the radiographs based upon periostial elevation, joint space narrowing, and deposition of calcium.

In the developing test, when the calcium salt of A87689 was administered orally at 25 mg/kg in a 1% CMC solution, it inhibited paw swelling by 59%. When the calcium salt of A87689 was administered intraperitoneally at 10 mg/kg in DMSO, it inhibited paw swelling by 98%.

When the sodium salt of A87689 was administered intraperitoneally in DMSO at 5 mg/kg inhibited paw swelling by 108%; however, peritonitis was evident in the rats receiving this treatment. Under the same conditions, but administered at 10 mg/kg, the sodium salt of A87689 inhibited paw swelling by 101% (two rats of this group died from peritonitis).

The "established test" is a variation of the "developing test" in which the all of the conditions are the same but treatments with compound are not started until day 14 or 15 when the rats have an established disease as indicated by at least 0.5 mL of swelling of the uninjected paw.

In the established test, A87689 calcium salt, when administered at 50 mg/kg in 1% CMC intraperitoneally, inhibited the paw swelling of the established lesion by 58%.

Tests with the A87689 compounds described in this specification have also demonstrated the activity of these compounds against human T-cell leukemia virus and HIV-1. The following is a description of the test systems used in analyzing the effectiveness at A87689 compounds against these diseases.

## In Vitro Moloney Murine Leukemia Virus (Mo-MuLV) Assay

A87689 compounds are tested in vitro against Moloney murine leukemia virus (Mo-MuLV, Clone E7, The Salk Institute, La Jolla, California) using an XC assay as described by Rowe, W. P., et al., Virol. 42: 1136-1139 (1970). Generally, 3-5 x $10^3$ SC-1 cells (CRL-1404; American Type Culture Collection (ATCC)) per well of a 96-well microtiter plate are seeded in minimal essential medium supplemented with 5% fetal bovine serum, penicillin (150 units/mL), streptomycin (150 µg/mL), and polybrene (2 µg/mL). Each well is infected with Mo-MuLV (multiplicity of infection = 10 PFU/cell) the next day.

After allowing the virus to adsorb to the cells for 1 hour, medium containing serial dilutions of A87689 compounds are added to the cells. No semi-solid medium is used.

After incubation for 5 days (when cells are confluent), the SC-1 cells are UV irradiated for 10 seconds and

$5\text{-}8 \times 10^4$ XC cells (CCL-165; ATCC) per well are added. Usually, two additional days of incubation are required to obtain full virus-induced cytopathic effect. The effect of A87689 compounds in this assay is reproduced in Table 4.

For assessment of virucidal activity of A87689 compounds, serial dilution of the compounds to be assessed are pre-incubated with Mo-MuLV for 1 hour at 37°C. SC-1 cells are then infected with two-fold dilutions of the various A87689 compound-virus mixtures. After allowing the virus to adsorb to the cells for 1 hour at 37°C, the virus-infected cells are washed three times with Hank's balanced salt solution (HBSS; GIBCO, Grand Island, N.Y.) and the cells are monitored for the appearance of cytopathic effect using the XC cell assay as described above.

## In Vitro HIV Antiviral Assay

The HTLV-IIIB strain of HIV-1 is propagated in the human T-lymphocyte cell line "H9"(Popovic, m., et al., Science, 224: 497-500 (1984)). The virus inoculum consists of supernat fluids from H9-IIIB producer cultures.

MT-2 (Harada, et al., Science, 229: 563-566 (1985)) and CEM (Nara, et al., Nature, 332: 469-470 (1988)) cell lines were used for routine screening. Cells are grown in RPMI 1640 medium. For propagation of H9 cells, the RPMI medium is supplemented with fetal bovine serum at 20% (v/v). The medium used to propagate the MT-2 cell line, for the dilution of compounds and for maintenance of cultures during the assay, is supplemented with 10% (v/v) fetal bovine serum. Each medium also contains 100 units/mL of penicillin, 100 mcg/mL streptomycin, and 25 mM HEPES buffer. Cultures are maintained in disposable tissue culture labware at 37°C in a humidified atmosphere of 5% $CO_2$ in air.

Pre-assay preparation also includes formulating the XTT-PMS reagent used for calorimetric evaluation of cell viability by mixing XTT (tetrazolium reagent; Diagnostic Chemicals, Oxford, CT.) at 200μg/mL and 0.02 mM of PMS (phenazine methosulfate; Sigma Chemical, St. Louis, MO.).

A87689 compounds are dissolved in DMSO at 40 mg/mL or in sterile, deionized water at 2 mg/mL. In particular, the calcium salt and the free acid of A87689 was dissolved in DMSO. The compound in DMSO is initially diluted in medium to a concentration of 0.2 mg/mL, whereas the compound in water is initially diluted in medium to a concentration of 0.2 mg/mL. Subsequent dilutions are made in log or 0.5 log series.

For evaluation of cytotoxicity, each dilution is added to microplates in the amount of 100 μL/well. A87689 compounds are tested in triplicate wells per dilution, with infected cells, and in duplicate wells per dilution with uninfected cells.

Prior to preparation of infected and uninfected cells, a viable cell count is performed on the cells to be used via trypan blue.

The desired total number of cells is placed in a 50 mL conical centrifuge tube (sterile and disposable), and virus is added to give a MOI of 0.03 $TCID_{50}$/cell for MT-2 cells, and approximately 0.12 $TCID_{50}$/cell for CEM cells. Fresh medium is added to adjust the cell density to $1 \times 10^5$ cells/mL, and the virus-cell suspension is incubated at 37°C for 1-2 hours prior to plating. Uninfected cells are prepared in the same manner, but without addition of virus.

Next, infected and uninfected cells are added to plates in the amount of 100 μL/well to give a starting cell number of $1 \times 10^4$ cells/well. Microplates prepared in this manner are then incubated for 7 days in a humidified atmosphere of 5% $CO_2$ in air.

For quantitation of viral cytopathic effect (CPE) and determination of activity of A87689 compounds, 50 μL of XTT-PMS solution are added to each well, and the microplates are returned to the $CO_2$ incubator for 4 hours. The lids are then removed from the plates and replaced with adhesive plate sealers. Plates are read with the plate sealers in place in a VMax Plate Reader at 450 nm and 650 nm. The effect of A87689 compounds in this assay is reported in Table 5.

## Table 6. Activity of A87689 Compounds in Moloney Murine Leukemia Virus In Vitro Assay

| A87689 | $IC_{50}$* |
|---|---|
| Calcium Salt | 0.4 µg/mL |
| Free Acid | 0.4 µg/mL |
| Penta-Sodium Salt | 0.5 µg/mL |
| Tetra-acetate Free Acid | 0.2 µg/mL |

*$IC_{50}$ = concentration of A87689 compound required to inhibit CPE by 50%.

## Table 7. Activity of A87689 Compounds in HIV In Vitro Assay

| A87689 | $IC_{50}$* |
|---|---|
| Free Acid | 8.5 µg/mL |
| Penta-Sodium Salt | 18.8 µg/mL |

*$IC_{50}$ = concentration of A87689 compound required to inhibit CPE by 50%.

The following examples further illustrate the compounds of the present invention. The examples are not intended to be limiting to the scope of the invention in any respect and should not be construed.

### Example 1

### Fermentation of A87689

A. Shake-flask

The culture A87689, NRRL 18815, either as a lyophilized pellet or as a suspension maintained in liquid nitrogen, is used to inoculate a vegetative medium having the composition of vegetative medium A:

## Vegetative Medium A

| Ingredient | Amount (%) |
|---|---|
| Glucose | 1.0 |
| Potato Dextrin | 3.0 |
| Soybean Flour | 2.0 |
| Cottonseed flour* | 2.0 |
| CaCO$_3$ | 0.2 |
| Tap H$_2$O | q.s. to 1 liter |

No pH adjustment

*PROFLO Flour, Traders Protein, P.O. Box 8407, Memphis, TN 38108

When the culture is maintained in liquid nitrogen, ampoules are prepared using mycelium from a vegetative culture (48 hour incubation, 30°C) which are suspended to culture volume in suspending medium. The suspending medium contains lactose (100 g), glycerol (200 mL), and deionized water (q.s. to 1 L).

A liquid nitrogen ampoule is used to inoculate 50 mL of vegetative medium in 250 mL flasks. The cultures are incubated at 30°C for 48 hours on a shaker orbiting in a two-inch (5.08 cm) circle at 250 rpm.

The vegetative culture is used (2% v/v inoculum) to inoculate 50 mL of a production medium having the following composition:

## Production Medium I

| Ingredient | Amount (%) |
|---|---|
| Glucose | 3.0 |
| Peptone * | 0.75 |
| Blackstrap Molasses | 0.5 |
| CaCO$_3$ | 0.2 |
| Deionized H$_2$O | q.s. to 1 liter |

No pH adjustment

* BACTO PEPTONE, Difco Laboratories

The inoculated production medium is incubated in 250 mL Erlenmeyer flasks at 30°C for 5 to 6 days on a shaker orbiting in a two-inch circle at 250 rpm.

B. Stirred Bioreactor Fermentation

In order to provide a larger volume of inoculum, 15 mL of incubated first-stage vegetative culture, prepared as described in Section A, is used to inoculate 600 mL of a second-stage vegetative medium having the same composition as that of the first-stage vegetative medium. The second-stage culture is incubated in a 2-L wide mouth Erlenmeyer flask for about 24 hours at 30°C on a shaker orbiting in a two-inch circle at 250 rpm.

Incubated second-stage vegetative culture (2.4 L) thus prepared is used to inoculate 80 to 115 liters of sterile production medium II.

## Production Medium II

| Ingredient | Amount (%) |
|---|---|
| Glucose | 2.35 |
| Peptone * | 0.75 |
| Blackstrap Molasses | 1.4 |
| CaCO$_3$ | 0.2 |
| Deionized H$_2$O | q.s. to 1 liter |

No pH adjustment; antifoam (SAG 471) is added to the medium to control foam

* BACTO PEPTONE

The inoculated production medium is allowed to ferment in a 165-L stirred bioreactor for 7 to 8 days at 30°C. The air flow and agitator speed in the stirred vessel are computer controlled to maintain a dissolved oxygen level at 45% of air saturation. pH is controlled (if needed) between 6.7 and 7.6, using H$_2$SO$_4$ and NaOH. Glucose solution (0.33 g/mL of deionized H$_2$O) is fed at a rate of 4.5 g/L/day starting at 25 to 27 hours after inoculation. The rate is increased to 5.7 g/L/day starting at 42 to 44 hours.

## Example 2

### Fermentation of A87689.1

Compound A87689 is produced according to the method of Example 1, but using the A87689.1 (NRRL 18851) culture.

## Example 3

### Isolation And Purification Of A87689

Fermentation broth (88L) (prepared as described in Example 2) was filtered through a ceramic filter (Membralox Systems, Illinois Water Treatment, Rockford, Illinois) to yield 66 L of filtrate containing A87689. The filtrate was pumped over a stainless steel column (3″ x 48″) of DIAION HP-20SS (a resin of the polystyrene type, Mitsubishi Chemical Industries, Ltd., Tokyo) (5 L) at a flow rate of 500 mL/minute. The column was washed with 20 L of methanol:water (1:3), and the activity was eluted with methanol:water (1:1), collecting 10 - 4-L fractions. The fractions were assayed for PLA$_2$ inhibitory activity. Fractions 4 and 5, which contained the majority of activity, were combined and concentrated in vacuo to 2500 mL as pool 1. Fractions 3,6,7 and 8 were combined as a second pool of activity. The precipitate which formed in pool 1 was centrifuged off and dissolved in 60 mL DMSO. A 10-mL aliquot of the DMSO solution was pumped onto a DYNAMAX HPLC C$_8$ column (4.1 x 30 cm) (Rainin Instruments Co., Emeryville, CA) containing 400 mL of silica gel C$_8$ (150A) resin. The column was developed with a gradient of acetonitrile:water:5% NH$_4$OAc (10:85:5) to acetonitrile: water:5% NH$_4$OAc (50:45:5) in 70 minutes with a flow rate of 25 mL/min. and collecting 25 mL fractions. The elution was monitored with a Waters 481 detector (Millipore Corporation, Milford, MA) at 254 nm. Crystals which formed in fractions 36, 37, and 38, were filtered off and dried in vacuo. The aliquot separation was repeated 5 more times to give a total yield of 1162 mg of crystalline A87689 salt (decomposed at about 300°C, no true melting point).

## Example 4

### Alternate Procedure for Isolation and Purification of A87689

Whole fermentation broth (225 L), obtained as described in Example 1, was filtered through a filter press with 3% HYFLO SUPERCEL filter aid (diatomaceous earth, Johns Manville Products Corp.). The filtrate (190 L) was adjusted from pH 7.4 to pH 7.0 with 5N HCl; DIAION HP-20 resin (20 L) was added and stirred for 2 hours. The resin was removed by filtration, washed with 100 L of distilled water, then washed with 100 L of water:methanol (3:1) by stirring in a vessel for 30 minutes. The supernate was decanted, and the resin was stirred with 50% aqueous methanol to elute the A87689 activity. The methanol supernate containing the activity was removed by filtration, and the resin was stirred with 100 L of methanol to remove the remainder of the activity and to regenerate the resin. The elution was monitored using the snake venom (Naja naja) PLA$_2$ assay.

The 50% methanol eluate was concentrated in vacuo to 20 L; pH was adjusted to 7.0; and the solution was extracted twice with 20 L of n-butanol. The spent aqueous layer was freeze-dried to yield 56 g of crude product. (The purification of this material, which was not n-butanol extractable, is described in Example 4B.) The n-BuOH extracts were combined and concentrated in vacuo to dryness. The residue was dissolved in 100 mL water and 100 mL dioxane and freeze dried to yield 14.7 g of crude A87689. The crude A87689 was dissolved in 300 mL methanol with sonication and allowed to stand at -10°C until a precipitate formed. The precipitate was filtered off and dried in vacuo to yield 1.4 g of partially purified A87689.

This material (100 mg) was stirred in 10 mL water, and the suspension was adjusted to pH 12.0 with 5N NaOH to dissolve the precipitate. The pH was slowly adjusted to 7.0 with 5N HCl, and the solution was applied to a DYNAMAX 150A column (Rainin Instrument Co., Emeryville, CA) containing 100 mL silica gel C$_8$ (150 A) resin. The column was developed with a gradient of acetonitrile:water:1% NH$_4$OAc (5:85:10) to acetonitrile:water:1% NH$_4$OAc (50:40:10) in 50 minutes at a flow rate of 8 mL/min. The elution was monitored at 260 nm using a Waters 481 detector (Millipore Corporation, Milford, MA). Fractions 2 and 3 were combined, concentrated in vacuo to remove the acetonitrile, and allowed to stand overnight for crystallization. This procedure was repeated, and the crystals from two runs were combined, filtered and dried in vacuo to Yield 29 mg of crystalline A87689 calcium salt (m.p. - decomposed above 300°C) This salt form had a very low solubility in water, but was soluble in DMSO and is called the "water insoluble salt" or the "calcium salt" in discussions of utility.

## Example 4A

### Additional Alternate Procedure for Isolation and Purification of A87689

An alternate procedure to Example 4 was to resuspend the partially purified A87689 dried precipitate in H$_2$O and apply the solution directly to the color without adjustment from pH 12 to pH 7. The resulting non-crystalline sodium salt was lyophilized and was soluble in water. In discussions of utility, this form is called the "water soluble salt" or the "sodium salt".

## Example 4B

### Purification of the N-Butanol Non-Extractable Preparation

A portion of the n-butanol non-extractable material from Example 4 (200 mg) was dissolved in 10 mL water and applied to a DYNAMAX 150A column containing 100 mL silica gel C$_8$ resin. The column was developed with a gradient of acetonitrile:0.1% NH$_4$OAc (1:20) to acetonitrile:0.1% NH$_4$OAc (1:1) in 30 minutes at a flow rate of 10 mL/min, collecting 8-mL fractions and monitoring the elution at 260 nm. Crystals which formed in fraction 22 (the most active fraction) were filtered off and dried in vacuo to yield 8 mg of crystalline A87689 salt. The filtrate was freeze-dried to yield an additional 19 mg of amorphous A87689 salt.

## Example 5

### Preparation of A87689 Free Acid from the A87689 Calcium Salt

Purified A87689 calcium salt (0.44 g), obtained as described in Example 4, was suspended in 5N HCl (0.05 L) at ambient temperature; acetone (0.05 L) was added. A yellow solution was observed after 10 minutes, and after 30 minutes precipitation began. The suspension was stirred 18 hours under nitrogen at ambient temperature. The reaction mixture was reduced to 1/2 volume in vacuo and filtered to give a yellow powder (0.58 g) after washing with IN HCl solution. The still wet powder was dissolved in $CHCl_3$ (0.2 L) and washed 2x with $H_2O$. The $CHCl_3$ layer was dried over $Na_2SO_4$. Filtration and removal of the solvent in vacuo gave a yellow foam (0.306 g) which was the free acid of A87689. The final material was yellow and crystalline. TLC (Merck SG) demonstrated 1-spot material ($R_f$=0.5; chloroform:methanol:ammonia, 6:3:1) detected by iodine vapor, and HPLC indicated 77.9% purity. The free acid had an HPLC retention time of 6.4 minutes, using a µBondapak $C_{18}$ SG column (Waters Chromatography Division, Millipore Corporation) in a gradient of $CH_3CN$:0.1% $NH_4OAc$ (1:9 to 7:3) solution at a flow rate of 2 mL/min, and detecting by U.V. at 254 nm.

The free acid of A87689 has the following characteristics:

Molecular weight: 1184.462

Empirical formula: $C_{66}H_{72}O_{20}$

FAB-MS (M+1) :

Found: 1185.4695

Calculated: 1185.4680

Elemental analysis:

Calculated: C, 66.88; H, 6.12

Found: C,66.52; H, 5.95. No nitrogen or sulfur wass detected.

```
UV  (EtOH,  lmax),:
Neutral:   304 (e=54805)      238 (e=64335)
Base:      300 (e=56569)      237 (e=72695)
Acid:      314 (e=59871)      236 (e=38628)
           218 (e=40085)
```

Melting point: Starts charring > 200 to 320°C Titration shows pKa of 3.5 (approximately three groups)

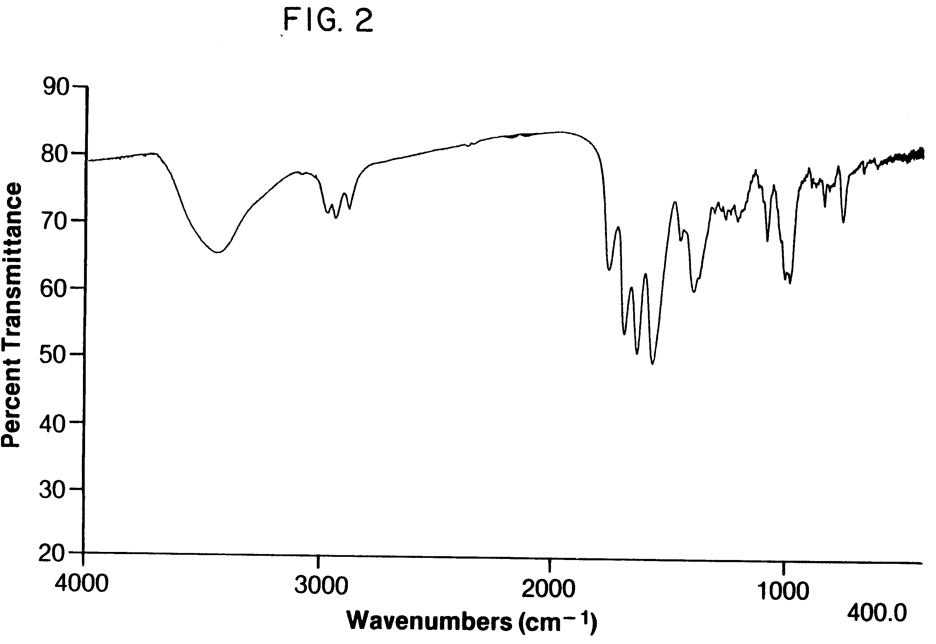

The IR spectrum of the free acid of A87689 (KBr pellet) is shown in Figure 2 of the accompanying drawings. The most significant absorption maxima occur at the following frequencies ($cm^{-1}$): 3439, 1758, 1688, 1636, 1568, 1395, 1002, 981 and 753.

## Example 6

### Preparation of the Tetra-acetate Calcium Salt

A87689 calcium salt (100 mg) was dissolved in anhydrous pyridine (3 mL). Acetic anhydride (1 mL) was added to this solution. The solution was heated to 40°C for 120 hours under nitrogen. The solution was cooled to room temperature, and concentrated to dryness in vacuo to give a tacky residue. The residue was triturated with chloroform: methanol (1:1), filtered, and dried in vacuo to yield a light yellow powder (112 mg). The A87689 tetra-acetate calcium salt had a retention time of 12.2 min. on the HPLC system described in Example 5. The tetra-acetate calcium salt has the following characteristics:

Molecular weight: 1390.460

Empirical formula: $C_{74}H_{78}O_{24}Ca$

FAB-MS (M+1) :

Found: 1391.4659

Calculated: 1391.4677

```
UV(EtOH,  lmax):
Neutral:  300 (e=31700)      238 (e=32700)
Base:     295 (e=57900)      206 (e=311000)
Acid:     306 (e=53900)      225 (E=62300)
```

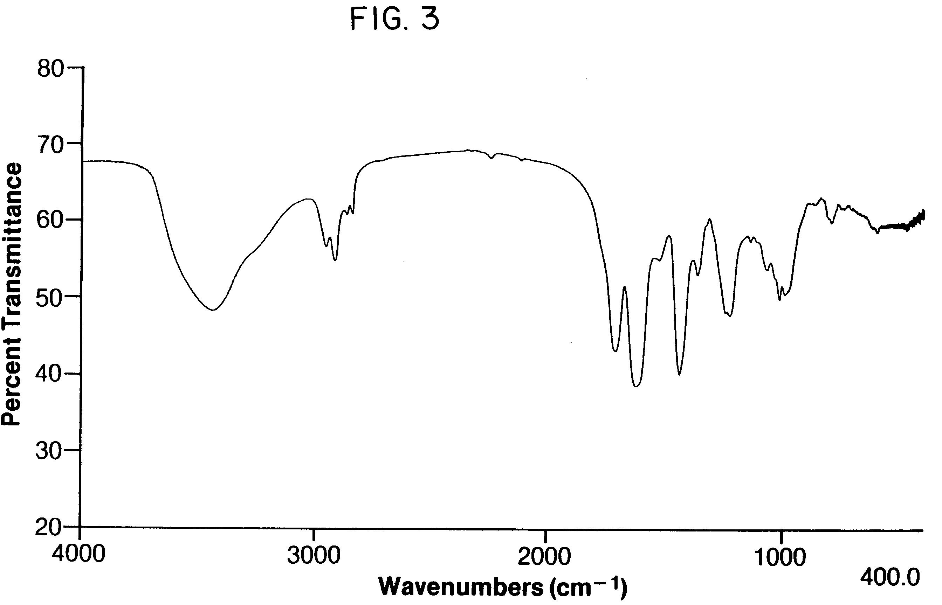

The IR spectrum of the tetra-acetate calcium salt of A87689 (KBr pellet) is shown in Figure 3 of the accompanying drawings- The most significant absorption maxima occur at the following frequencies ($cm^{-1}$): 3436, 1728, 1641, 1457, 1241, 1027, and 1003.

## Example 7

### Preparation of the A87689 Tetramethyl Ether Calcium Salt

A87689 calcium salt (0.1 g) was dissolved in DMSO (10 mL) containing 10% NaOH (1.5 mL). To this solution, methyl iodide (0.5 mL) was added. This solution was stirred at 40°C for 18 hours under nitrogen. It was cooled to room temperature and adjusted to pH 1.5 with 1 N HCl. The precipitate was collected by filtration, washed 2x with $H_2O$, and dried in vacuo to give 115 mg of tan powder.

The A87689 tetramethyl ether calcium salt had an $R_f$ value of 0.75 on the TLC system described in Example 5. The A87689 tetramethyl ether calcium salt has the following additional characteristics:

Molecular weight:    1280
Empirical formula:    $C_{70}H_{78}O_{20}Ca$

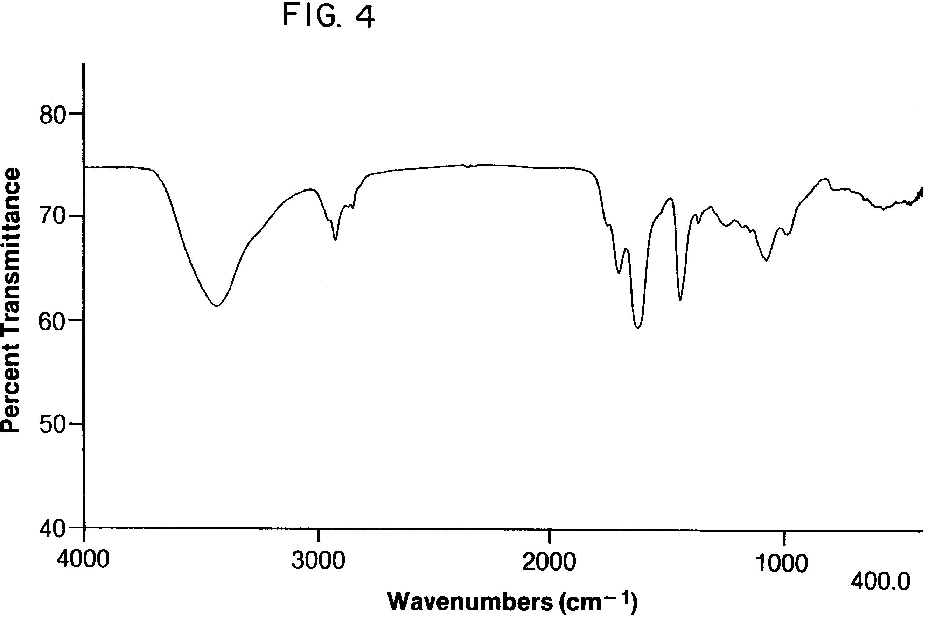

The IR spectrum of the tetramethyl ether calcium salt of A87689 (KBr) pellet is shown in Figure 4 of the accompanying drawings. The most significant absorption maxima occur at the following frequencies ($cm^{-1}$): 3434, 1718, 1637, 1465 and 1089.

## Example 8

### Preparation of the A87689 Penta-Sodium Salt

A87689 free acid (70 mg) was dissolved in MeOH (1 mL), to which was added 1 mL of $H_2O$, followed by an adjustment of the pH to 12 with 2N NaOH. This solution was stirred for one hour at room temperature under nitrogen. The solution was concentrated in vacuo to near dryness to give semi-solids, which were triturated with 2 mL of acetonitrile:methanol (1:1) and filtered to give a white powder (52 mg). This water-soluble powder was triturated with methanol, and the resulting solids were collected and dried to give a white solid (38 mg).

The A87689 penta-sodium salt had an $R_f$ value of 0.3 on the TLC system described in Example 5 and a retention time of 5.25 on the HPLC system described in Example 5. The A87689 penta-sodium salt has the following additional characteristics:

Molecular weight: 1294.371
Empirical formula: $C_{66}H_{67}O_{20}Na_5$
FAB-MS(M+1):
    Found: 1295.3814
    Calculated: 1295.3792

```
UV  (EtOH,  1max)
Neutral:   299  (e=40379)        237  (e=47771)
Base:      299  (e=42623)        235  (e=30162)
Acid:      312  (e=40908)        235  (e=30162)
           221  (e=29110)
```

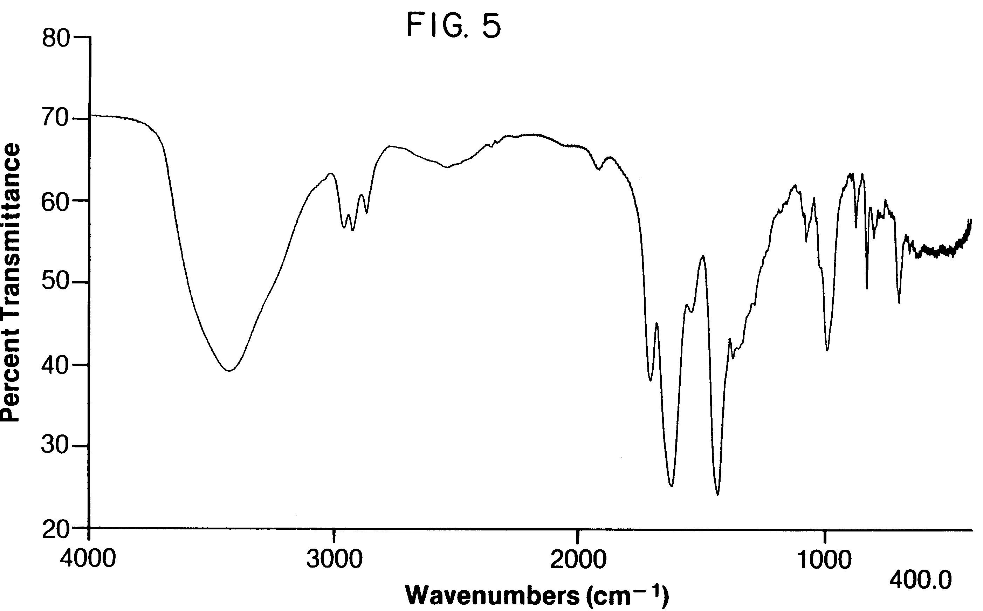

The IR spectrum of the penta-sodium salt of A87689 (KBr pellet) is shown in Figure 5 of the accompanying drawings. The most significant absorption maxima occur at the following frequencies ($cm^{-1}$): 3430, 1710, 1623, 1436, 996, 834 and 701.

## Example 9

### Preparation of the A87689 Tetramethyl Ether Free Acid

A87689 calcium salt (50 mg) was dissolved in 5 mL DMSO containing 0.8 mL of 10% NaOH solution. To this was added 0.25 mL methyl iodide. This reaction was stirred at 40°C for 18 hours under nitrogen. The reaction was then cooled to room temperature, and the pH was adjusted to 2 with 5N HCl. The reaction mixture was then concentrated to 0.75 the original volume under vacuum and diluted with 100 mL $CHCl_3$. The $CHCl_3$ layer was washed 2x with 5% $Na_2S_2O_3$ followed by 2 washes with water. The organic layer was removed and dried under vacuum to yield 68 mg creamy-yellow solids. The solids were triturated with petroleum ether B and then with diethyl ether. The solids were collected by filtration and dried under vacuum to yield 33 mg of a fine yellow residue.

The A87689 tetramethyl ether free acid had an $R_f$ value of 0.7 on the TLC system described in Example 5 and a retention time of 10.23 minutes on the HPLC system described in Example 5. The A87689 tetramethyl ether free acid has the following additional characteristics:

Molecular Weight: 1240

Empirical formula: $C_{70}H_{80}O_{20}$

FAB-MS(M + 1):

Found: 1241.5387

Calculated: 1241.5322

```
UV(EtOH, lmax)
Neutral:   304  (e=47104)         239  (e=51704)
Base:      296  (e=51763)         233  (e=71752)
           212  (e=227629)
Acid:      312  (e=50737)         233  (e=37297)
           218  (e=37705)
```

## Claims

1. The use of quartromicin in the preparation of a medicament useful for inhibiting $PLA_2$.

2. A87689, as herein before described- or a $C_{1-6}$-alkyl ether or $C_{1-6}$-alkanoyl ester derivative of A87689, or a pharmaceutically acceptable salt thereof.

3. A compound as claimed in Claim 2 which is A87689 or a pharmaceutically acceptable salt thereof.

4. The calcium salt of A87689.

5. The free acid of A87689.

6. The penta-sodium salt of A87689.

7. A process for preparing A87689 or a derivative thereof as claimed in any one of Claims 2 to 6, which comprises cultivating Amycolatopsis mediterranei NRRL 18815. NRRL 18851, or an A87689-producing mutant thereof, in a culture medium containing assimilable sources of carbon, nitrogen and inorganic salts under submerged aerobic fermentation conditions; optionally followed by salifying, esterifying, acylating, or forming an ether derivative thereof, as appropriate.

8. A biologically pure culture of Amycolatopsis mediterranei NRRL 18815, NRRL 18851, or an A87689-producing mutant thereof.

9. An A87689 compound as claimed in any one of Claims 2 to 6 for use as an inhibitor of $PLA_2$.

10. An A87689 compound as claimed in any one of Claims 2 to 6 for use as an agent for the management of retroviral infections.

11. An A87689 compound as claimed in any one of Claims 2 to 6 for use as an agent for the management of HIV.

12. An A87689 compound as claimed in any one of Claims 2 to 6 for use as an agent for the management of AIDS.

13. A pharmaceutical formulation comprising as an active ingredient an A87689 compound as claimed in any one of Claims 2 to 6, associated with one or more pharmaceutically acceptable carriers, excipients or diluents therefor.

**Claims for the following Contracting States: GR, ES**

1. A process for preparing A87689, as herein before described, or a $C_{1-6}$-alkyl ether or $C_{1-6}$-alkanoyl ester derivative of A87689, or a pharmaceutically acceptable salt thereof, which comprises cultivating Amycolatopsis mediterranei NRRL 18815 , NRRL 18851, or an A87689-producing mutant thereof, in a culture medium containing assimilable sources of carbon- nitrogen and inorganic salts under submerged aerobic fermentation conditions; optionally followed by salifying, esterifying, acylating, or forming an ether derivative thereof, as appropriate.

2. A process according to Claim 1 for preparing A87689 or a pharmaceutically acceptable salt thereof.

3. A process according to Claim 1 for preparing the calcium salt of A87689.

4. A process according to Claim 1 for preparing the free acid of A87689.

5. A process according to Claim 1 for preparing the penta-sodium salt of A87689.

6. A process according to Claim 1 for preparing the tetra-methyl ether derivative of A87689.

7. A process according to Claim 1 for preparing the tetra-acetate derivative of A87689.

8. A biologically pure culture of Amycolatopsis mediterranei NRRL 18815, NRRL 18851, or an A87689-producing mutant thereof.

9. A process for preparing a pharmaceutical formulation which comprises admixing A87689, as herein before described, or a $C_{1-6}$-alkyl ether or $C_{1-6}$-alkanoyl ester derivative of A87689 or a pharmaceutically acceptable salt thereof, with one or more pharmaceutically acceptable carriers, diluents or excipients therefor.

# FIG. I

EP 0 526 258 A2

FIG. 2

# FIG. 3

EP 0 526 258 A2

# FIG. 4

# FIG. 5

EP 0 526 258 A2

## FIG. 6